(19) Europäisches Patentamt / European Patent Office / Office européen des brevets

(11) **EP 3 563 218 B1**

(12) **FASCICULE DE BREVET EUROPEEN**


(45) Date de publication et mention de la délivrance du brevet:
**06.01.2021 Bulletin 2021/01**

(21) Numéro de dépôt: **17832294.7**

(22) Date de dépôt: **22.12.2017**

(51) Int Cl.:
***G06F 3/01*** *(2006.01)* ***G06F 3/038*** *(2013.01)*
*A61B 5/04* *(2006.01)* *A61B 5/0478* *(2006.01)*
*A61B 5/048* *(2006.01)*

(86) Numéro de dépôt international:
**PCT/FR2017/053818**

(87) Numéro de publication internationale:
**WO 2018/122518 (05.07.2018 Gazette 2018/27)**


(54) **PROCÉDÉ ITÉRATIF DE CALIBRATION D'UNE INTERFACE NEURONALE DIRECTE**

ITERATIVES VERFAHREN ZUR KALIBRIERUNG EINER DIREKTEN NEURALEN SCHNITTSTELLE

ITERATIVE PROCESS FOR CALIBRATING A DIRECT NEURAL INTERFACE


(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.12.2016 FR 1663473**

(43) Date de publication de la demande:
**06.11.2019 Bulletin 2019/45**



(73) Titulaire: **Commissariat à l'énergie atomique et aux énergies alternatives**
**75015 Paris (FR)**

(72) Inventeur: **YELISYEYEV, Andriy**
**10034 New York, NY (US)**

(74) Mandataire: **INNOV-GROUP**
**310, avenue Berthelot**
**69372 Lyon Cedex 08 (FR)**


(56) Documents cités:
**US-B2- 9 480 583**


Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).





Printed by Jouve, 75001 PARIS (FR)

## Description

### DOMAINE TECHNIQUE

L'invention concerne une interface neuronale directe, usuellement désignée par l'acronyme anglosaxon BCI, signifiant Brain Computer Interface, pour permettre la commande d'un effecteur automatisé, par exemple un robot ou un exosquelette, par des signaux électrophysiologiques corticaux.

### ART ANTERIEUR

Le domaine des interfaces neuronales directes est en plein développement et apparaît comme une solution séduisante pour permettre à des personnes handicapées de commander des effecteurs par la pensée. Il s'agit d'enregistrer, généralement à l'aide d'électrodes corticales, des signaux électrophysiologiques. Ces derniers sont traités par des algorithmes permettant d'extraire un signal de commande, pour commander des effecteurs. Le signal de commande peut permettre le pilotage d'un exosquelette, d'un ordinateur ou d'un robot, pour fournir une assistance à l'utilisateur. Les algorithmes mis en œuvre permettent une traduction d'une instruction donnée par l'utilisateur, cette instruction étant captée, par les électrodes, sous la forme de signaux dits électrophysiologiques, car représentatifs de l'activité électrique des neurones. Cette activité électrique est généralement mesurée au niveau du cortex, au moyen d'électrodes corticales disposées dans la boîte crânienne. Elle peut également être mesurée par des électrodes d'électroencéphalographie, moins intrusives car disposées sur le scalp, mais également moins performantes, notamment au niveau de la résolution spatiale. Une autre solution est d'enregistrer les signaux électrophysiologiques par magnétoencéphalographie, ce qui nécessite une installation dédiée.

Les algorithmes mis en œuvre sont généralement basés sur un modèle prédictif. Le modèle prédictif utilise des données d'entrée, obtenues par un prétraitement des signaux électrophysiologiques enregistrés. Les données d'entrée sont généralement multidimensionnelles, et comprennent :

- une composante spatiale, représentative de l'origine spatiale du signal électrophysiologique ;
- une composante fréquentielle, représentative de l'intensité du signal électrophysiologique dans différentes bandes de fréquence ;
- une composante temporelle, représentative de l'évolution temporelle du signal électrophysiologique, chaque signal électrophysiologique étant acquis selon une fréquence d'échantillonnage élevée, typiquement quelques kHz, durant un intervalle temporel d'acquisition de quelques centaines de millisecondes à quelques secondes. L' intervalle temporel d'acquisition est généralement désigné par le terme anglosaxon "Epoch", signifiant "époque".

Ces différentes composantes forment un bloc de données multidimensionnelles, comportant un grand nombre de données, qu'il faut traiter en temps réel ou en quasi temps réel pour obtenir un signal de sortie représentatif d'une action commandée par l'utilisateur. Les données mesurées, à chaque intervalle temporel d'acquisition, peuvent être regroupées sous la forme d'un tenseur de dimension 3, dit tenseur d'entrée, chaque dimension correspondant à une composante évoquée ci-dessus. Compte tenu du nombre important de données à traiter, des modèles prédictifs basés sur des algorithmes de réduction du nombre de données ont été mis en œuvre. Le brevet US9480583 décrit l'application d'une méthode de régression linéaire des moindres carrés partiels multivariée permettant d'établir un signal de commande à partir de signaux électrophysiologiques enregistrés. Une telle méthode est connue sous l'acronyme anglosaxon "NPLS" ou par le terme "N-way Partial Least Squares". L'application d'une telle méthode a également été décrite dans la publication Eliseyev A, Aksenova T (2013) "Recursive N-way Partial Least Squares for Brain Computer Interface" PIOS ONE july 2013, Volume 8 Issue 7 e69962. Une telle méthode est également décrite dans le document Yelisyeyev A "Brain-Computer Interface with cortical electrical activity recording" Human health and pathology,

Université de Grenoble, 2011. Cette méthode permet de former un modèle prédictif en se basant sur une phase de calibration. La phase de calibration est réalisée sur la base d'un tenseur de calibration d'entrée, comportant des données représentatives de signaux électrophysiologiques mesurées durant différentes époques, dites données d'entrée de calibration. A chaque époque correspondent des données de sortie, représentatives du signal de commande de l'effecteur ou d'un état de l'effecteur, les données de sortie sur toutes les époques formant des données de sortie de calibration.

Les inventeurs ont constaté que du fait de la variabilité du cerveau, la durée de validité d'un modèle prédictif est limitée et qu'il est nécessaire de procéder à des mises à jour régulières. Cela implique la mise en œuvre de phases de calibration espacées dans le temps, de façon à acquérir des données de calibration. Une telle mise à jour peut nécessiter la manipulation d'un nombre de données très important, combinant les données d'une calibration antérieure et les données d'une nouvelle calibration. Ils ont proposé un procédé de calibration, permettant une mise à jour du modèle de prédiction, tout en mettant en œuvre une quantité raisonnable de données. On réduit alors la puissance de calcul nécessaire à la mise à jour, ainsi que la durée de la mise à jour.

## EXPOSE DE L'INVENTION

Un premier objet de l'invention et un procédé de calibration d'une interface neuronale directe, l'interface étant apte à acquérir des signaux électrophysiologiques et à former un tenseur d'entrée à partir des signaux électrophysiologiques acquis, pour établir, à l'aide d'un modèle prédictif, un tenseur de sortie, destiné à commander un effecteur, le modèle prédictif étant obtenu par une calibration, la calibration comportant les étapes suivantes :

a) acquisition de signaux électrophysiologiques de calibration dits d'entrée pour obtenir un tenseur dit de calibration d'entrée, chaque signal électrophysiologique étant représentatif d'une activité neuronale durant la calibration ;
b) acquisition de signaux de calibration dits de sortie pour obtenir un tenseur, dit de calibration de sortie, chaque signal de calibration de sortie étant représentatif d'un signal de commande de l'effecteur ou d'un état de l'effecteur durant la calibration, les signaux de calibration de sortie étant acquis, de préférence, simultanément aux signaux électrophysiologiques de calibration d'entrée ;
c) calcul d'un tenseur de covariance, représentant une covariance du tenseur de calibration d'entrée;
d) calcul d'un tenseur de covariance croisée représentant une covariance du tenseur de calibration d'entrée et du tenseur de calibration de sortie;
e) application d'une régression multivariée des moindres carrés partiels à partir du tenseur de covariance obtenu à l'étape c) et du tenseur de covariance croisée obtenu lors de l'étape d) de façon à obtenir un modèle prédictif, ainsi que des paramètres de calibration;

les étapes a) à e) étant mises en œuvre lors d'une période de calibration courante, le procédé étant tel qu'après une première période de calibration,

- l'étape c) comporte une prise en compte du tenseur de covariance calculé au cours d'une période de calibration antérieure, et pondéré par un facteur d'oubli ;
- l'étape d) comporte une prise en compte du tenseur de covariance croisé calculé au cours de la période de calibration antérieure, et pondéré par le facteur d'oubli;
- l'étape e) comporte une prise en compte de paramètres de calibration résultant de la période de calibration antérieure.

Le facteur d'oubli peut être un réel positif strictement compris entre 0 et 1.

Lors de l'étape e), les paramètres de calibration peuvent comporter un jeu de vecteurs de projection, de telle sorte qu'après la première période de calibration, l'étape e) comporte une prise en compte d'un jeu de vecteurs de projection résultant de la calibration antérieure.

Lors de l'étape e) la régression multivariée des moindres carrés partiels peut être effectuée selon plusieurs itérations, à chaque itération correspondant un rang d'itération, chaque itération générant un modèle, dite modèle prédictif, permettant d'estimer un tenseur, dit tenseur de sortie, à partir d'un tenseur d'entrée,

- le tenseur d'entrée étant formé à partir de signaux électrophysiologiques acquis à différents instants ;
- le tenseur de sortie étant formé de données de sorties destinées à former des signaux de commande pour commander l'effecteur.

Le modèle prédictif peut comporter une matrice dite de prédiction.

Le procédé peut comporter une détermination d'un rang d'itération dit optimal, la détermination étant réalisée selon les étapes suivantes :

- prise en compte d'un tenseur de calibration d'entrée et d'un tenseur de calibration de sortie correspondant à la période de calibration courante ;
- prise en compte de modèles prédictifs respectivement associés à différents rangs d'itération de la régression multivariée effectuée durant la période de calibration antérieure ;
- application de chaque modèle prédictif au tenseur de calibration d'entrée de la période de calibration courante, de façon à obtenir une estimation du tenseur de calibration de sortie de la période de calibration courante, chaque estimation étant associée au rang de l'itération correspondant au modèle prédictif à partir de laquelle l'estimation est effectuée ;
- comparaison, pour chaque rang d'itération, de chaque estimation obtenue lors de l'étape précédente, au tenseur de calibration de sortie de la période de calibration courante;
- détermination d'un rang d'itération, dit optimal, en fonction de chaque comparaison. Le rang d'itération optimal est notamment celui pour lequel l'estimation est la plus proche du tenseur de calibration de sortie.

Un autre objet de l'invention est un procédé de commande d'un effecteur par une interface neuronale directe, comportant les étapes suivantes :

i) acquisition de signaux électrophysiologiques produits dans le cortex d'un individu ;
ii) traitement des signaux électrophysiologiques, pour former des données d'entrée, rassemblées selon un tenseur d'entrée;
iii) application d'un modèle prédictif au tenseur d'entrée de façon à estimer un tenseur de sortie;
iv) formation d'un signal de commande de l'effecteur à partir du tenseur de sortie ;

le procédé étant caractérisé en ce que lors de l'étape iii) le modèle prédictif est déterminé par une calibration réalisée selon un procédé de calibration tel que décrit dans la demande.
Le tenseur de sortie peut correspondre à un état de l'effecteur souhaité par l'utilisateur générant les signaux électrophysiologiques. Dans ce cas, les signaux de commande permettent de configurer l'effecteur selon l'état ainsi estimé.
Lors de l'étape iii), on peut appliquer le modèle prédictif correspondant au rang d'itération optimal (*F**) préalablement déterminé.
Un troisième objet de l'invention est une interface neuronale directe, comportant :

- des capteurs, aptes à acquérir des signaux électrophysiologiques représentatifs d'une activité corticale ;
- un processeur, pour traiter lesdits signaux électrophysiologiques ;
- un effecteur, configuré pour être actionné par un signal de commande généré par le processeur ;
- le processeur étant configuré pour mettre en oeuvre les étapes ii) à iv) d'un procédé selon le deuxième objet de l'invention, à partir des signaux électrophysiologiques acquis par les capteurs. Les capteurs peuvent être des électrodes corticales, des électrodes d'électroencéphalographie ou des capteurs de magnétoencéphalographie.

D'autres avantages et caractéristiques ressortiront plus clairement de la description qui va suivre de modes particuliers de réalisation de l'invention, donnés à titre d'exemples non limitatifs, et représentés sur les figures listées ci-dessous.

## FIGURES

La figure 1 montre une représentation de l'interface neuronale.
La figure 2A représente les principales étapes d'un procédé de calibration.
La figure 2B représente les principales étapes d'un procédé d'estimation utilisant la calibration.
La figure 2C représente les principales étapes d'un procédé de détermination d'un nombre optimal d'itérations.
La figure 3 représente un exemple de détermination d'un rang optimal d'itérations.
La figure 4 représente les principales sous-étapes d'une régression linéaire multivariée des moindres carrés partiels.

## EXPOSE DE MODES DE REALISATION PARTICULIERS

La figure 1 représente les principaux éléments d'une interface neuronale 1 selon l'invention. Il s'agit d'un dispositif comportant des capteurs 2, permettant l'acquisition de signaux électrophysiologiques représentatifs d'une activité neuronale. Les capteurs 2 sont par exemple des électrodes corticales. Les capteurs 2 sont reliés à un processeur 3, par une liaison filaire ou sans fil. Le processeur 3 est apte à exécuter des instructions codées dans une mémoire 4. Ces instructions comprennent notamment les algorithmes décrits ci-après. En mettant en œuvre ces instructions, le processeur génère un signal de commande destiné à piloter un effecteur 5.
L'effecteur 5 est un actionneur apte à effectuer une action sous l'effet du signal de commande qui lui est adressé. Il peut s'agir d'un exosquelette, d'un robot, ou d'un ordinateur. L'effecteur est alors commandé par les signaux électrophysiologiques acquis par les électrodes corticales 2, via un algorithme codé dans la mémoire 4 et mis en oeuvre par le processeur 3. Le dispositif 1 est une interface neuronale directe, dans le sens où il permet le contrôle de l'effecteur 5 par les signaux électrophysiologiques mesurés par les capteurs 2. Les signaux électrophysiologiques peuvent être acquis par des électrodes corticales placées au contact du cortex, des capteurs de magnétoencéphalographie ou des électrodes d'éléctroencéphalographie.
Comme indiqué en lien avec l'art antérieur, le signal de commande est formé par le processeur 3 à partir des signaux électrophysiologiques, regroupés, après prétraitement, selon un tenseur, dit tenseur d'entrée, en mettant en œuvre un modèle prédictif. Le modèle prédictif génère un tenseur de sortie, regroupant des données, dites données de sortie, permettant le contrôle de l'effecteur 5. Le modèle prédictif fait l'objet d'un apprentissage, ou calibration, durant lequel le signal de commande, dit signal de sortie, est maîtrisé.

Dans cet exemple, les signaux électrophysiologiques $S_1...S_{I_3}$ ,générés par un individu, sont acquis par un nombre $I_3$ d'électrodes $E_1...E_{I_3}$ corticales réparties à la surface du cortex dudit individu. $I_3$ est par exemple égal à 64. Chaque signal électrophysiologique est représentatif d'une activité corticale. Les signaux électrophysiologiques sont acquis selon une fréquence d'échantillonnage de 1 KHz et sont rassemblés par blocs de données d'entrée $\underline{\boldsymbol{x}}(n)$ d'une durée glissante égale à 1 seconde, chaque bloc correspondant à une fenêtre d'observation, ou époque (traduction du terme anglais "epoch"). Chaque bloc de données est décalé du bloc suivant et du bloc précédent de 100 ms. Les signaux électrophysiologiques acquis font l'objet d'un prétraitement avant d'être regroupés selon un tenseur d'entrée.

Chaque signal électrophysiologique acquis par une électrode fait par exemple l'objet d'une analyse fréquentielle selon $I_2$ bandes de fréquence. Par exemple, $I_2$=15. Les bandes de fréquence sont par exemple comprises entre 10 kHz et 150 kHz. L'analyse fréquentielle peut être par exemple une transformée en ondelettes.

Chaque signal électrophysiologique peut également faire l'objet d'une décimation, de façon à s'étendre sur $I_1$ incréments temporels. Par exemple, $I_1$ = 10.

Les signaux électrophysiologiques, après prétraitement (analyse fréquentielle et décimation) sont regroupés en des blocs de données d'entrée. Chaque bloc de données d'entrée $\underline{\boldsymbol{x}}(n)$ , correspondant à un instant $n$, est tridimensionnel, et de dimension $I_1 \times I_2 \times I_3$. Les blocs de données d'entrée $\underline{\boldsymbol{x}}(n)$ peuvent être regroupés en un tenseur $\underline{\boldsymbol{X}}$, dit tenseur d'entrée, regroupant un nombre N blocs de données $\underline{\boldsymbol{x}}(n)$ d'entrée consécutifs. Le tenseur d'entrée est de dimension $N \times I_1 \times I_2 \times I_3$.

A chaque bloc de donnée d'entrée $\underline{\boldsymbol{x}}(n)$ correspond un signal de commande $\underline{\boldsymbol{y}}(n)$, représentant la commande à adresser à des effecteurs à l'instant $n$. Comme précédemment indiqué, un modèle prédictif permet, à partir du tenseur d'entrée $\underline{\boldsymbol{X}}$, d'obtenir un tenseur dit de sortie $\underline{\boldsymbol{Y}}$.

Le tenseur de sortie regroupe N blocs de données $\underline{\boldsymbol{y}}(n)$ de sortie consécutifs, de telle sorte que chaque bloc de données d'entrée $\underline{\boldsymbol{x}}(n)$ est associé à un bloc de données de sortie $\underline{\boldsymbol{y}}(n)$, le bloc de données de sortie permet la formation du signal de commande d'un ou plusieurs effecteurs. Le tenseur de sortie peut correspondre à un état de l'effecteur, par exemple une position de l'effecteur, tel que souhaité par l'individu générant les signaux électrophysiologiques. Les signaux de commandes sont alors adaptés pour configurer l'effecteur selon cet état.

La dimension de chaque bloc de données de sortie peut varier selon les cas de figure. Dans l'exemple considéré, chaque bloc de sortie pilote trois articulations d'un bras, en l'occurrence le poignet, le coude et l'épaule, avec trois degrés de liberté par articulation. Chaque bloc de sortie $\underline{\boldsymbol{y}}(n)$ est donc une matrice 3 x 3, correspondant à l'état des trois articulations.

On décrit à présent l'établissement d'un modèle prédictif selon l'invention, en lien avec la figure 2A. Le modèle prédictif est réalisé au cours d'une phase de calibration, au cours de laquelle le signal de sortie, c'est-à-dire le signal de commande de l'effecteur 5, ou l'état de l'effecteur 5, est maîtrisé. Par exemple, on demande à l'individu d'effectuer un mouvement particulier, durant lequel on acquiert des signaux électrophysiologiques. Ainsi, les blocs de données de calibration d'entrée $\underline{\boldsymbol{x}}(n)$ sont mesurés alors que chaque bloc de données de calibration de sortie $\underline{\boldsymbol{y}}(n)$, c'est-à-dire l'état de l'effecteur 5, est également mesuré ou connu.

Les signaux de calibration de sortie sont soit mesurés, soit imposés, mais ils sont connus, contrairement à la phase d'utilisation de l'interface neuronale directe, au cours de laquelle les signaux de sortie sont estimés à partir des signaux électrophysiologiques d'entrée. Les signaux de calibration de sortie sont représentatifs d'un signal de commande de l'effecteur, ou d'un état de l'effecteur, durant la calibration. Par exemple, on impose ou on mesure une trajectoire à l'effecteur, auquel cas les données de calibration de sortie représentent les différentes positions de l'effecteur durant la trajectoire, chaque position correspondant à un état de l'effecteur. On peut également observer le comportement de l'individu et l'actionnement volontaire, par ce dernier, de l'effecteur, auquel cas les données de calibration de sortie correspondent au signal de commande de l'effecteur ou à l'état de l'effecteur (actionné ou non actionné). Par exemple, comme décrit dans le brevet US9480583, l'effecteur est une pédale et le signal de calibration de sortie représente l'actionnement de la pédale par l'individu.

Les données de calibration d'entrée constituent un tenseur d'entrée dit de calibration $\underline{\boldsymbol{X}}^{(t)}$ tandis que les données de calibration de sortie forment un tenseur de calibration de sortie $\underline{\boldsymbol{Y}}^{(t)}$. $t$ désigne une période temporelle, dite période de calibration, à laquelle les données d'entrée et de sortie de calibration sont acquises, les données de calibration d'entrée et de sortie étant de préférence acquises simultanément.

Chaque bloc de calibration d'entrée $\underline{\boldsymbol{x}}(n)$ a pour dimension $I_1 \times ... \times I_k$ : chacune de ses coordonnées est notée $x_{n,I_1 \times ... \times I_k}$. Dans cet exemple, $k$ = 3.

Chaque bloc de de calibration sortie $\underline{\boldsymbol{y}}(n)$ a pour dimension $J_1 \times ... \times J_m$ : chacune de ses coordonnées est notée $y_{n,J_1 \times ... \times J_m}$. Dans cet exemple, $m$ = 2.

Etape 100 : constitution des tenseurs de calibration $\underline{\boldsymbol{X}}^{(t)}$ et $\underline{\boldsymbol{Y}}^{(t)}$. Les tenseurs de calibration peuvent comporter un nombre N de blocs de données de calibration d'entrée et de sortie de calibration $\underline{\boldsymbol{x}}(n)$ et $\underline{\boldsymbol{y}}(n)$.

La dimension du tenseur de calibration d'entrée $\underline{\boldsymbol{X}}^{(t)}$ est $N \times I_1 \times ... \times I_k$.

La dimension du tenseur de calibration de sortie $\underline{\boldsymbol{Y}}^{(t)}$ est : $N \times J_1 \times ... \times J_m$.

Une des particularités de la calibration est qu'après une première période de calibration, $t > 1$, elle prend en compte les tenseurs de calibration antérieurs $\underline{\boldsymbol{X}}^{(t-1)}$ et $\underline{\boldsymbol{Y}}^{(t-1)}$, sous la forme de tenseurs de covariance et de covariance croisée. Autrement dit, lors d'une première calibration, correspondant à $t = 1$, l'algorithme de calibration est mis en œuvre sur la base des premiers tenseurs de calibration $\underline{\boldsymbol{X}}^{(t=1)}$ et $\underline{\boldsymbol{Y}}^{(t=1)}$ Lors des calibrations suivantes, on prend en compte des tenseurs de calibration, dits courants, acquis à une période t, dite période courante, durant laquelle chaque calibration est effectuée. Ces calibrations prennent en compte des tenseurs de covariance établis à partir des tenseurs de calibration antérieurs $\underline{\boldsymbol{X}}^{(t-1)}$, $\underline{\boldsymbol{Y}}^{(t-1)}$obtenus à une période de calibration antérieure à la période courante. La contribution des tenseurs de calibration antérieurs est toutefois pondérée par un facteur d'oubli $\lambda$, comme décrit en lien avec l'étape 140.

Etape 110 : normalisation : les tenseurs de calibration $\underline{\boldsymbol{X}}^{(t)}$ et $\underline{\boldsymbol{Y}}^{(t)}$ sont préférentiellement normalisés, de telle sorte que chacun de leurs termes soit centré et réduit par rapport à une valeur moyenne et à une déviation standard. Pour cela, on détermine un nombre d'observations dit effectif : $N^{(t)} = \lambda N^{(t-1)}+N^{(t)}$ et on calcule une somme pondérée $S^t_{x_{i_1,\dots,i_k}}$ et une somme quadratique pondérée $S^{2t}_{x_{i_1,\dots,i_k}}$ respectivement selon les expressions :

$$S^t_{x_{i_1,\dots,i_k}} = \lambda \sum_{n=1}^{N^{(t)}-1} x^{t-1}_{n,i_1,\dots,i_k} + \sum_{n=1}^{N^{(t)}} x^t_{n,i_1,\dots,i_k} \; ; (1)$$

$$S^{2t}_{x_{i_1,\dots,i_k}} = \lambda \sum_{n=1}^{N^{(t)}-1} \left(x^{t-1}_{n,i_1,\dots,i_k}\right)^2 + \sum_{n=1}^{N^{(t)}} \left(x^t_{n,i_1,\dots,i_k}\right)^2 \; ; (2)$$

où $x^t_{n,i_1,\dots,i_k}$ désigne chaque terme du tenseur de calibration d'entrée $\underline{\boldsymbol{X}}^{(t)}$

Lors de la première calibration ($t = 1$), le premier terme de chaque expression est nul.

On peut alors établir une valeur moyenne $\mu^t_{x_{i_1,\dots,i_k}}$ et une déviation standard $\sigma^t_{x_{i_1,\dots,i_k}}$ des termes $x^t_{n,i_1,\dots,i_k}$ composant le tenseur d'entrée $\underline{\boldsymbol{X}}^{(t)}$ selon les expressions :

$$\mu^t_{x_{i_1,\dots,i_k}} = \frac{S^t_{x_{i_1,\dots,i_k}}}{N^{(t)}} \;(3) \text{ et } \sigma^t_{x_{i_1,\dots,i_k}} = \sqrt{\frac{S^{2t}_{x_{i_1,\dots,i_k}} - \frac{\left(S^t_{x_{i_1,\dots,i_k}}\right)^2}{N^{(t)}}}{N^{(t)}-1}} \;(4)$$

De façon identique, on peut établir une moyenne et une déviation standard des termes $y^t_{n,j_1,\dots,j_m}$ du tenseur de sortie $\underline{\boldsymbol{Y}}^{(t)}$ selon les expressions :

$$\mu^t_{y_{j_1,\dots,j_m}} = \frac{S^t_{y_{j_1,\dots,j_m}}}{N^{(t)}} \;(5) \text{ et } \sigma^t_{y_{j_1,\dots,j_m}} = \sqrt{\frac{S^{2t}_{y_{j_1,\dots,j_m}} - \frac{\left(S^t_{y_{j_1,\dots,j_m}}\right)^2}{N^{(t)}}}{N^{(t)}-1}} \;(6)$$

La normalisation consiste à centrer et réduire chaque terme $x^t_{n,i_1,\dots,i_k}$ et $y^t_{n,j_1,\dots,j_m}$ de telle sorte que :

$$x^t_{n,i_1,\dots,i_k} = \frac{x^t_{n,i_1,\dots,i_k} - \mu^t_{x_{i_1,\dots,i_k}}}{\sigma^t_{x_{i_1,\dots,i_k}}} \;(7)$$

$$y^t_{n,j_1,\dots,j_m} = \frac{y^t_{n,j_1,\dots,j_m} - \mu^t_{y_{j_1,\dots,j_m}}}{\sigma^t_{y_{j_1,\dots,j_m}}} \;(8)$$

Etape 120 : calcul du tenseur de covariance et du tenseur de covariance croisée. Le tenseur de covariance est :

$$\underline{C_{XX}}^{(t)} = \underline{X}^{(t)} \times_1 \underline{X}^{(t)} \ (10)$$

$$\underline{C_{XY}}^{(t)} = \underline{X}^{(t)} \times_1 \underline{Y}^{(t)} \ (11)$$

$\times_1$ désigne le produit de tenseur selon le mode 1, décrit dans la littérature, notamment dans la thèse mentionnée dans l'art antérieur.

Etape 130 : prise en compte d'une calibration précédente.

Les tenseurs de covariance et de covariance croisée sont modifiés, par une prise en compte de tenseurs de covariance et de covariance croisée relatifs à une période antérieure de calibration, cette période antérieure étant notée $t$ - 1.

$$\underline{C_{XX}}^{(t)} = \lambda \underline{C_{XX}}^{(t-1)} + \underline{X}^{(t)} \times_1 \underline{X}^{(t)} \ (12)$$

$$\underline{C_{XY}}^{(t)} = \lambda \underline{C_{XY}}^{(t-1)} + \underline{X}^{(t)} \times_1 \underline{Y}^{(t)} \ (13)$$

Cette étape est une étape clef du procédé, car elle permet la prise en compte de données utilisées lors d'une phase précédente de calibration, la prise en compte de cet historique étant pondérée par un facteur d'oubli $\lambda$. Le facteur d'oubli $\lambda$ est un réel strictement compris entre 0 et 1.

Ainsi, lorsqu'une calibration est réalisée à une période de calibration courante $t$, elle tient compte d'une calibration précédente, réalisée à une période de calibration antérieure $t$ - 1. Le procédé permet ainsi d'effectuer une mise à jour de calibrations, chaque calibration prenant en compte une calibration effectuée antérieurement. La prise en compte de l'historique est pondérée par le facteur d'oubli $\lambda$. Plus il est faible, moins l'historique est pris en compte.

Lors de la première période de calibration, ($t$ = 1), $\underline{C_{XX}}^{(t=1)} = \underline{X}^{(t=1)} \times_1 \underline{X}^{(t=1)}$ et $\underline{C_{XY}}^{(t=1)} = \underline{X}^{(t=1)} \times_1 \underline{Y}^{(t=1)}$.

Etape 140 : mise à jour du modèle prédictif

Au cours de cette étape, le modèle prédictif est mis à jour par application d'un algorithme connu sous l'acronyme NPLS (N-Partial Least Squares), signifiant régression multivariée des moindres carrés partiels, aux tenseurs de covariance et de covariance croisés résultant de l'étape 130. L'algorithme suivi est un algorithme itératif, selon les principes décrits dans la publication Dayal B. "Improved PLS Algorithms", Journal of Chemometrics, Vol. 11, 73-85 (1997), et plus précisément l'algorithme modifié numéro 2 annexé à ladite publication. Cet algorithme itératif permet d'obtenir, à chaque itération $f$ :

- un ensemble de vecteurs de projection, $\boldsymbol{w_1^f}, \dots \boldsymbol{w_k^f}$. Le vecteur $\boldsymbol{w_1^f}$ est de dimension $I_1$, le vecteur $\boldsymbol{w_2^f}$ est de dimension $I_2$...le vecteur $\boldsymbol{w_k^f}$ est de dimension $I_k$. L'ensemble des itérations permet de former des ensembles de vecteurs de projection $\left\{\boldsymbol{w_1^f}, \dots \boldsymbol{w_k^f}\right\}_{f=1}^{F} {}^{t}$ correspondant à la période de calibration $t$. Ces vecteurs de projection permettent une projection du tenseur de calibration d'entrée dans un espace de variable latente.
- une matrice de prédiction $\boldsymbol{\tilde{B}^f}$, formant le modèle prédictif, permettant une estimation $\hat{\underline{Y}}$ d'un tenseur de sortie $\underline{Y}$ à partir d'un tenseur d'entrée $\underline{X}$. L'ensemble des itérations permet de former un ensemble de matrices de prédiction $\left\{\boldsymbol{\tilde{B}^f}\right\}_{f=1}^{F} {}^{t}$ correspondant à la période de calibration t.
- un tenseur dit de biais $\underline{\boldsymbol{Y}_0^f}$, formant également le modèle prédictif. L'ensemble des itérations permet de former un ensemble de tenseurs de biais $\left\{\underline{\boldsymbol{Y}_0^f}\right\}_{f=1}^{F} {}^{t}$ correspondant à la période de calibration $t$.

$f$ est le rang de l'itération. Il s'agit d'un entier compris entre 1 et un nombre d'itération maximal $F$, ce dernier étant par exemple égal à 200.

Le détail de cette étape, et l'obtention des grandeurs listées ci-dessus, sont décrits ultérieurement, en lien avec la figure 4 et les étapes 141 à 149 décrites ci-après.

Suite à cette étape, on dispose d'un modèle prédictif permettant l'estimation $\underline{\hat{Y}}^f$ d'un tenseur de sortie $\underline{Y}$ à partir d'un tenseur d'entrée $\underline{X}$ selon l'expression :

$$\underline{\widehat{Y}}^f = \tilde{B}^f \underline{X} + \underline{Y}_0^f \quad (14)$$

La notation $\underline{\hat{Y}}^f$ désigne le fait que le tenseur $\underline{\hat{Y}}$ est estimé selon le modèle prédictif, en l'occurrence la matrice $\tilde{B}^f$ et le tenseur de biais $\underline{Y}_0^f$ définis au cours de l'itération *f*.

On remarque que contrairement à l'art antérieur, les tenseurs d'entrée et de sortie permettant la mise en oeuvre de l'algorithme NPLS ne sont pas des tenseurs d'entrées $\underline{X}$ et $\underline{Y}$ respectivement obtenus avec des blocs de données de calibration d'entrée et de sortie mesurés, comme décrit dans le brevet US9480583. L'invention met en œuvre :

- le tenseur $\underline{C_{XX}}^{(t)}$, qui exprime une covariance du tenseur de calibration d'entrée $\underline{X}^{(t)}$, de dimension $I_1 \times ... \times I_k \times I_1 \times ... \times I_k$;
- le tenseur $\underline{C_{XY}}^{(t)}$, qui exprime une covariance croisée du tenseur de calibration d'entrée $\underline{X}^{(t)}$ et du tenseur de calibration de sortie $\underline{Y}^{(t)}$, de dimension $I_1 \times ... \times I_k \times J_1 \times ... \times J_m$.

La dimension des tenseurs sur la base desquels la calibration itérative est réalisée est donc réduite par rapport à l'art antérieur, dans lequel les tenseurs de calibration d'entrée et de sortie sont respectivement $\underline{X}^{(t)}$ et $\underline{Y}^{(t)}$, dont les dimensions respectives sont $N \times I_1 \times ... \times I_k$ et $N \times J_1 \times ... \times J_m$.

L'étape 140 est réalisée en incrémentant le rang d'itération *f* jusqu'à l'atteinte d'un rang d'itération maximal *F*.

Etape 150 : fin d'algorithme. On dispose alors d'un modèle prédictif correspondant à chaque rang d'itération *f*, calibré sur la base des tenseurs de calibration $\underline{X}^{(t)}$ et $\underline{Y}^{(t)}$, la calibration étant réalisée à l'aide d'un tenseur de calibration antérieur $\underline{X}^{(t-1)}$ et $\underline{Y}^{(t-1)}$, et plus précisément à l'aide des tenseurs de covariance et de covariance croisée résultant de la calibration antérieure.

Le modèle de calibration prédictif peut être mis à jour sur la base de nouveaux tenseurs de calibration $\underline{X}^{(t+1)}$ et $\underline{Y}^{(t+1)}$, en réitérant les étapes 100 à 150, en incrémentant la période de calibration t.

La fréquence de mise à jour est variable. Les inventeurs ont constaté qu'il est préférable de réaliser, initialement, différentes calibrations successives, en considérant des tenseurs de calibration dits initiaux $\underline{X}^{ini}$ et $\underline{Y}^{ini}$ de grande dimension, avec $N = 7000$. De tels tenseurs de calibration sont acquis selon une durée de calibration initiale longue, pouvant par exemple durer plus de 10 minutes. En effet, du fait de la décimation, chaque incrément temporel est espacé de 100 ms. Une durée d'acquisition $N = 7000$ correspond à 7000 blocs de données $\underline{x}(n)$ et $\underline{y}(n)$, acquis selon une durée d'acquisition glissante de 1s, chaque bloc étant décalé du bloc précédent ou suivant de 100 ms. Au cours de la calibration initiale, chaque tenseur $\underline{X}^{ini}$ et $\underline{Y}^{ini}$ est segmenté en 70 segments $\underline{X}^{(t=1)}.....\underline{X}^{(t=70)}$ et $\underline{Y}^{(t=1)}.....\underline{Y}^{(t=70)}$, ou buffer, chaque segment comportant $N = 100$ blocs de données de calibration d'entrée et de sortie $\underline{x}(n)$ et $\underline{y}(n)$.

Chaque segment $\underline{X}^{(t)}$ et $\underline{Y}^{(t)}$ constitue respectivement un tenseur de calibration d'entrée et de sortie. Ainsi, les étapes 100 à 150 ci-dessous sont répétées successivement, en considérant chaque tenseur de calibration $\underline{X}^{(t)}$ et $\underline{Y}^{(t)}$ avec $1 \leq t \leq 70$.

A l'issue de ces 70 calibrations itératives successives, on dispose d'un modèle de prédiction robuste, pouvant être régulièrement mis à jour par exemple quotidiennement ou plusieurs fois par jours, par une séquence de calibration courte, nécessitant peu de blocs de données $\underline{x}(n)$ et $\underline{y}(n)$, par exemple $N = 100$, ce qui, compte tenu de la durée de chaque bloc (1 s) et du décalage entre chaque bloc (100 ms), correspond à une durée de 10 secondes. Pendant la calibration, l'utilisateur effectue des mouvements prédéterminés, de façon à maîtriser l'état de l'effecteur de sortie.

Entre chaque calibration, le modèle prédictif est utilisé de façon à estimer un état du tenseur de sortie $\underline{Y}$ correspondant à un tenseur d'entrée $\underline{X}$ en mettant en œuvre l'expression de prédiction (14). Les principales étapes, décrites sur la figure 2B, sont :

Etape 200 : acquisition de signaux électrophysiologiques, prétraitement, obtention de blocs de données d'entrée $\underline{x}(n)$ pour former le tenseur d'entrée $\underline{X}$.

Etape 210 : normalisation du tenseur d'entrée, comme décrit en lien avec l'étape 110.

Etape 220 : estimation du tenseur de sortie, $\underline{\hat{Y}}^f$ en appliquant l'expression (14). L'utilisation de l'expression (14) nécessite le choix d'un rang d'itération *f*. Ce choix peut être arbitraire, en étant défini, généralement une fois pour toutes, sur la base d'essais préliminaires. Le tenseur de sortie ainsi estimé permet d'obtenir des signaux de commandes pour commander l'effecteur 5. Par exemple, le tenseur de sortie comporte un état de l'effecteur souhaité par l'utilisateur, et les signaux de commande formés permettent d'aboutir à cet état.

Les inventeurs ont constaté que lors de l'utilisation du modèle prédictif, c'est-à-dire lors de l'estimation d'un tenseur de sortie $\hat{\underline{\boldsymbol{Y}}}^{f}$ sur la base d'un tenseur mesuré $\underline{\boldsymbol{X}}$, il est possible de déterminer un rang d'itération *f* optimal, ce nombre optimal étant noté *F**.

On décrit ci-après les principales étapes de la détermination du nombre optimal *F**, en lien avec la figure 2C. Pour cela, on applique les matrices de prédiction $\{\widetilde{\boldsymbol{B}}^{f}\}_{f=1}^{F\ \ t-1}$, obtenues lors d'une calibration antérieure, par exemple la période de calibration précédente *t* - 1, à des tenseurs de calibration $\underline{\boldsymbol{X}}^{(t)}$ et $\underline{\boldsymbol{Y}}^{(t)}$ correspondant à une période de calibration *t*.

Etape 300 : initialisation : *f* = 1

Etape 310 : estimation $\hat{\underline{\boldsymbol{Y}}}^{f(t)}$ de $\underline{\boldsymbol{Y}}^{(t)}$ en utilisant une matrice de prédiction $\widetilde{\boldsymbol{B}}^{f(t-1)}$ obtenue lors de la calibration précédente : $\hat{\underline{\boldsymbol{Y}}}^{f(t)} = \underline{\boldsymbol{X}}^{(t)}\widetilde{\boldsymbol{B}}^{f\,(t-1)} + \boldsymbol{Y}_{\boldsymbol{0}}^{f}$

Etape 320 : calcul d'une erreur correspondant au rang d'itération *f*, selon l'expression:

$$e_f^t = \gamma e_f^{t-1} + Error(\hat{\underline{\boldsymbol{Y}}}^{f(t)},\ \underline{\boldsymbol{Y}}^{(t)})$$

$Error(\hat{\underline{\boldsymbol{Y}}}^{f}(\boldsymbol{t}),\boldsymbol{Y}(\boldsymbol{t}))$ représente une erreur d'estimation de $\underline{\boldsymbol{Y}}^{(t)}$, par exemple une erreur quadratique.

Lorsque t = 1 (suite à la première calibration), $e_f^{t=1} = Error(\hat{\underline{\boldsymbol{Y}}}^{f(t=1)},\ \underline{\boldsymbol{Y}}^{(t=1)})$. *y* est un facteur d'oubli, indépendant du facteur d'oubli $\lambda$ considéré lors des étapes 100 à 150, strictement compris entre 0 et 1.

Etape 330 : incrémentation de *f* jusqu'à *f* = *F*.

Etape 340 : détermination de *F**, tel que $F^* = \underset{f}{\operatorname{argmin}}\, e_f^t.$

Selon cet algorithme, le rang d'itération optimal *F**. est ajusté après chaque mise à jour du modèle prédictif, c'est-à-dire après chaque période de calibration. Cet algorithme utilise les tenseurs de calibration d'entrée et de sortie correspondant à une période de calibration courante *t*. Il consiste à obtenir une estimation du tenseur de calibration de sortie $\underline{\boldsymbol{Y}}^{(t)}$, à la période courante, en appliquant un modèle prédictif, $(\{\widetilde{\boldsymbol{B}}^{f}\}_{f=1}^{F\ \ t-1},\{\underline{\boldsymbol{Y}}_{\boldsymbol{0}}^{f}\}_{f=1}^{F\ \ t})$ issu d'une période de calibration antérieure *t* - 1, au tenseur de calibration d'entrée à ladite période courante $\underline{\boldsymbol{X}}^{(t)}$. Cela permet d'améliorer la précision de l'estimation du tenseur de sortie lors de l'application du modèle de prédiction, selon l'expression (14). Ainsi, durant les étapes de prédiction, suivant la calibration à la période courante *t*, l'estimation du tenseur de sortie est effectuée selon l'expression :

$$\hat{\underline{\boldsymbol{Y}}}^{f=F^*} = \widetilde{\boldsymbol{B}}^{f=F^*}\underline{\boldsymbol{X}} + \underline{\boldsymbol{Y}}_{\boldsymbol{0}}^{f=F^*} \quad (15)$$

Les inventeurs ont testé expérimentalement l'algorithme de détermination du rang d'itération *F**. optimal après 70 calibrations, chaque calibration étant réalisée sur un buffer de 100 blocs de données (*N* = 100). Pour cela, on a utilisé des signaux de calibration mesurés sur un singe, par une matrice de 64 électrodes corticales. Les signaux de calibration mesurés par les électrodes sont traités, de façon à former le tenseur de calibration d'entrée $\underline{\boldsymbol{X}}^{(t)}$ tel que précédemment décrit. Les mouvements des 3 articulations d'un bras du singe ont été enregistrés et forment le tenseur de calibration de sortie $\underline{\boldsymbol{Y}}^{(t)}$.

La figure 3 représente l'évolution du nombre d'itération *F**. optimal en fonction des différentes calibrations, chaque calibration correspondant à une abscisse variant entre 1 et 70. Chaque calibration est effectuée en prenant un nombre maximal d'itérations *F* égal à 200. On observe que le nombre optimal d'itérations *F**. tend vers 57.

La figure 4 montre les principales sous-étapes de l'étape 140 de mise à jour itérative du modèle prédictif. Ces étapes sont mises en oeuvre itérativement, avec un rang d'itération *f* compris entre 1 et *F* = 200.

Les données d'entrées sont les tenseurs de calibration d'entrée $\underline{\boldsymbol{X}}^{(t)}$ et de sortie $\underline{\boldsymbol{Y}}^{(t)}$, ainsi que des vecteurs de projection $\{\boldsymbol{w}_{\boldsymbol{1}}^{f}, \dots \boldsymbol{w}_{\boldsymbol{k}}^{f}\}_{f=1}^{F\ \ t-1}$ obtenus suite à une calibration antérieure. Lors de la première période de calibration *t* = 1, ces vecteurs sont unitaires.

Sous-étape 141 : Initialisation : les matrices internes **P**, **Q** et **R,** définies ci-après, sont initialisées à 0. $\mathbf{P} \in \mathbb{R}^{(I_1 \ldots I_k)\times F}$; $\mathbf{Q} \in \mathbb{R}^{(J_1 \ldots J_m)\times F}$; $\mathbf{R} \in \mathbb{R}^{(I_1 \ldots I_k)\times F}$

On forme des matrices $C_{XX}{}^{(t)} \in \mathbb{R}^{(I_1\times\ldots\times I_k)\times(I_1\times\ldots\times I_k)}$ à partir du tenseur $\underline{C_{XX}}^{(t)}$ et une matrice $C_{XY}{}^{(t)}, \in \mathbb{R}^{(I_1\times\ldots\times I_k)\times(J_1\times\ldots\times J_m)}$ à partir du tenseur $\underline{C_{XY}}^{(t)}$.

Les étapes 142 à 149 sont ensuite effectuées de façon itérative, *f* étant le rang d'itération.

Sous-étape 142 : Calcul de vecteurs propres. Si *m* > 1 (*m* est l'ordre du tenseur de sortie), on détermine le vecteur propre $\tilde{\mathbf{q}}$ correspondant à la valeur propre la plus élevée d'une matrice constituée par le produit des matrices $C_{XX}{}^{(t)T} \times C_{XY}{}^{(t)}$. ***T*** désigne l'opérateur transposée. Si *m* = 1, le vecteur considéré est $\tilde{\mathbf{q}} = C_{XY}{}^{(t)}$

Sous-étape 143 : Formation d'un tenseur $\underline{C}$ à partir d'une matrice ***C*** formée par un produit matriciel $C_{XY}{}^{(t)}\mathbf{q}.: \underline{C} = C_{XY}{}^{(t)}\,\tilde{\mathbf{q}}.\ \underline{C} \in \mathbb{R}^{(I_1\times\ldots\times I_k)}$

Sous-étape 144 : Décomposition par analyse factorielle parallèle du tenseur ***C*** formé lors de l'étape 143 en considérant les vecteurs de projection $\left\{\boldsymbol{w}_1^f, \ldots\ \boldsymbol{w}_n^f\right\}^{t-1}$ d'une calibration antérieure, ou les vecteurs de projection initialisés à 1 lors de la première calibration.

Sous-étape 145 : formation d'un tenseur $\underline{\boldsymbol{w}} = \boldsymbol{w}_1^f \circ \ldots \circ \boldsymbol{w}_n^f$ et formation d'un vecteur ***w*** $\in \mathbb{R}^{(I_1\ldots I_k)}$ à partir du tenseur $\underline{\boldsymbol{w}}$. Le vecteur *w* est ensuite normalisé par rapport à sa norme.

Sous-étape 146 : formation du vecteur de régression.

On pose ***r*** = ***w*** et on met à jour le vecteur de régression ***r*** par ***r*** = ***r*** - **[*P*(:, *u*)*ᵀwR*(:, *u*)]** en faisant varier *u* entre 1 et *f*-1.

Sous-étape 147 : détermination de vecteurs internes **p** et **q**, et mise à jour de matrices internes ***P*** et ***Q*** et ***R***.

$$\mathbf{p} = \frac{\left(\boldsymbol{r}^T C_{XX}{}^{(t)}\right)^T}{\boldsymbol{r}^T C_{XX}{}^{(t)}\boldsymbol{r}} \text{ et } \mathbf{q} = \frac{\left(\boldsymbol{r}^T C_{XY}{}^{(t)}\right)^T}{\boldsymbol{r}^T C_{XX}{}^{(t)}\boldsymbol{r}}$$

***P***(:, *f*) = **p; *Q***(:, *f*) = **q; *R***(:,*f*) = **r**

Sous-étape 148 : Déflation.

$$C_{XY}{}^{(t)} = C_{XY}{}^{(t)} - \left(\boldsymbol{r}^T C_{XX}{}^{(t)}\boldsymbol{r}\right)\mathbf{p}\,\mathbf{q}^T$$

Sous-étape 149 : Mise à jour des matrices formation de la matrice de prédiction $B^f$:

$\boldsymbol{B^f} = \boldsymbol{R}(:,1{:}f)\boldsymbol{Q}(:,1{:}f)^{\boldsymbol{T}}$

La matrice de prédiction peut faire l'objet d'une normalisation. chaque terme $b^f_{i_1,\ldots,i_k,j_1\ldots j_m}$ de la matrice $\boldsymbol{B^f}$ peut être normalisé de telle sorte que :

$$\tilde{b}^f_{i_1,\ldots,i_k,j_1\ldots j_m} = b^f_{i_1,\ldots,i_k,j_1\ldots j_m}\,\frac{\sigma^t_{y_{j_1,\ldots,j_m}}}{\sigma^t_{x_{i_1,\ldots,i_k}}}$$

$\sigma^t_{y_{j_1,\ldots,j_m}}$ et $\sigma^t_{x_{i_1,\ldots,i_k}}$ étant respectivement définis dans les équations (6) et (4).

Chaque coefficient $\tilde{b}^f_{i_1\ldots i_k,j_1\ldots j_m}$ forme la matrice de prédiction $\tilde{\boldsymbol{B}}^f$ du modèle prédictif mis en œuvre dans l'expression de prédiction (14) ou (15).

Par ailleurs, l'algorithme génère le tenseur de biais $\underline{\boldsymbol{Y}}^f_{\boldsymbol{0}}$, dont chaque terme $y^f_{0_{j_1,\ldots j_m}}$ est défini par l'expression :

$y^f_{0_{j_1,..j_m}} = \mu^t_{y_{j_1,...,j_m}} - \sum_{i_1...i_k,} \mu^t_{x_{i_1,...,i_k}} \tilde{b}^f_{i_1...i_k,j_1...j_m}$, les grandeurs $\mu^t_{y_{j_1,...,j_m}}$ et $\mu^t_{x_{i_1,...,i_k}}$ étant définies dans les expressions (4) et (3).

Les données de sortie de l'algorithme sont celles décrites en lien avec l'étape 140.

## Revendications

**1.** Procédé de calibration d'une interface neuronale directe (1), l'interface étant apte à acquérir des signaux électrophysiologiques ($S_1...S_{I_3}$) et à former un tenseur d'entrée **($\underline{X}$)**, à partir des signaux électrophysiologiques acquis, pour établir, à l'aide d'un modèle prédictif, un tenseur de sortie **($\underline{Y}$)**, destiné à commander un effecteur (5), le modèle prédictif étant obtenu par une calibration, la calibration comportant les étapes suivantes :

a) acquisition de signaux électrophysiologiques de calibration dits d'entrée pour obtenir un tenseur **($\underline{X}^{(t)}$)** dit de calibration d'entrée, les signaux électrophysiologiques de calibration étant représentatifs d'une activité neuronale durant la calibration ;
b) acquisition de signaux de calibration dits de sortie pour obtenir un tenseur, dit de calibration de sortie **($\underline{Y}^{(t)}$)**, chaque signal de calibration de sortie étant représentatif d'un signal de commande de l'effecteur (5) ou d'un état de l'effecteur (5) durant la calibration;
c) calcul d'un tenseur de covariance **($\underline{C_{XX}}^{(t)}$**, représentant une covariance du tenseur de calibration d'entrée **($\underline{X}^{(t)}$)**;
d) calcul d'un tenseur de covariance croisée **($\underline{C_{XY}}^{(t)}$)** représentant une covariance du tenseur de calibration d'entrée **($\underline{X}^{(t)}$)** et du tenseur de calibration de sortie **($\underline{Y}^{(t)}$)**;
e) application d'une régression multivariée des moindres carrés partiels à partir du tenseur de covariance obtenu à l'étape c) et du tenseur de covariance croisée obtenu lors de l'étape d) de façon à obtenir un modèle prédictif $(\{\underline{\tilde{B}}^f\}_{f=1}^{F\ t}, \{\underline{Y}^f_0\}_{f=1}^{F\ t})$, ainsi que des paramètres de calibration $(\{w^f_1, ... w^f_k\}_{f=1}^{F\ t})$;

les étapes a) à e) étant mises en œuvre lors d'une période de calibration courante (t), le procédé étant tel qu'après une première période de calibration ($t$ = 1),

- l'étape c) comporte une prise en compte du tenseur de covariance **($\underline{C_{XX}}^{(t-1)}$)** calculé au cours d'une période de calibration antérieure (t - 1), et pondéré par un facteur d'oubli ($\lambda$);
- l'étape d) comporte une prise en compte du tenseur de covariance croisée **($\underline{C_{XY}}^{(t-1)}$)** calculé au cours de la période de calibration antérieure ($t$ - 1), et pondéré par le facteur d'oubli ($\lambda$);
- l'étape e) comporte une prise en compte de paramètres de calibration $\left(\{w^f_1, ... w^f_k\}_{f=1}^{F\ t-1}\right)$ résultant de la période de calibration antérieure.

**2.** Procédé selon la revendication 1, dans lequel le facteur d'oubli ($\lambda$) est un réel positif strictement compris entre 0 et 1.

**3.** Procédé selon la revendication 1 ou la revendication 2, dans lequel lors de l'étape e), les paramètres de calibration $\left(\{w^f_1, ... w^f_k\}_{f=1}^{F\ t}\right)$, comportent un jeu de vecteurs de projection de telle sorte qu'après la première période de calibration, l'étape e) comporte une prise en compte d'un jeu de vecteurs de projection $(\{w^f_1, ... w^f_k\}_{f=1}^{F\ t-1})$ résultant de la calibration antérieure.

**4.** Procédé selon l'une quelconque des revendications précédentes, dans lequel lors de l'étape e) la régression multivariée des moindres carrés partiels est effectuée selon un nombre (F) prédéfini maximal d' térations, à chaque itération correspondant un rang d'itération ($f$), chaque itération générant un modèle prédictif **($\underline{\tilde{B}}^f$)**, le modèle prédictif permettant d'estimer un tenseur **($\underline{\hat{Y}}^f$)**, dit tenseur de sortie, à partir d'un tenseur d'entrée ($\underline{X}$),

- le tenseur d'entrée $(\underline{\boldsymbol{X}})$ étant formé à partir de signaux électrophysiologiques acquis à différents instants ;
- le tenseur de sortie $(\underline{\hat{Y}}^f)$ comportant des données de sortie destinées à former des signaux de commande pour commander l'effecteur (5).

**5.** Procédé selon la revendication 4, comportant une détermination d'un rang d'itération dit optimal ($F^*$), la détermination étant réalisée selon les étapes suivantes :

- prise en compte d'un tenseur de calibration d'entrée $(\underline{\boldsymbol{X}}^{(t)})$ et d'un tenseur de calibration de sortie $(\underline{\boldsymbol{Y}}^{(t)})$ correspondant à la période de calibration courante ($t$);
- prise en compte des modèles prédictifs $(\{\tilde{\boldsymbol{B}}^f\}_{f=1}^{F\ \ t-1})$ respectivement associés à différents rangs d'itération ($f$) de la régression multivariée des moindres carrés partiels effectuée durant la période de calibration ($t$ - 1) antérieure ;
- application de chaque modèle prédictif $(\tilde{\boldsymbol{B}}^{f(t-1)})$ au tenseur de calibration d'entrée de la période de calibration courante $(\underline{\boldsymbol{X}}^{(t)})$, de façon à obtenir une estimation $(\underline{\hat{\boldsymbol{Y}}}^{f(t)})$ du tenseur de calibration de sortie de la période de calibration courante, chaque estimation étant associée au rang de l'itération ($f$) correspondant au modèle prédictif à partir duquel l'estimation est effectuée ;
- comparaison, pour chaque rang d'itération ($f$), de chaque estimation $(\underline{\hat{\boldsymbol{Y}}}^{f(t)})$ obtenue lors de l'étape précédente, au tenseur de calibration de sortie de la période de calibration courante $(\underline{\boldsymbol{Y}}^{(t)})$;
- détermination d'un rang d'itération ($F^*$), dit optimal, en fonction de chaque comparaison.

**6.** Procédé de commande d'un effecteur par une interface neuronale directe, comportant les étapes suivantes :

i) acquisition de signaux électrophysiologiques ($S_1...S_{I3}$) produits dans le cortex d'un individu ;
ii) traitement des signaux électrophysiologiques, pour former des données d'entrée, rassemblées selon un tenseur d'entrée $(\underline{\boldsymbol{X}})$;
iii) application d'un modèle prédictif $(\tilde{\boldsymbol{B}}^f, \tilde{\boldsymbol{B}}^{f=F^*})$ au tenseur d'entrée $(\underline{\boldsymbol{X}})$ de façon à estimer un tenseur de sortie $(\underline{\hat{\boldsymbol{Y}}}^f, \underline{\hat{\boldsymbol{Y}}}^{f=F^*})$;
iv) formation d'un signal de commande de l'effecteur (5) à partir du tenseur de sortie ;

le procédé étant **caractérisé en ce que** lors de l'étape iii) le modèle prédictif est déterminé par une calibration réalisée selon un procédé de calibration objet de l'une quelconque des revendications 1 à 5.

**7.** Procédé de commande selon la revendication 6, dans lequel lors de l'étape iii), on applique le modèle prédictif $(\tilde{\boldsymbol{B}}^{f=F^*})$ correspondant au rang d'itération optimal ($F^*$) déterminé selon le procédé de calibration selon la revendication 5.

**8.** Interface neuronale directe (1), comportant :

- des capteurs (2), aptes à acquérir des signaux électrophysiologiques ($S_1...S_{I3}$) représentatifs d'une activité corticale ;
- un processeur (3), pour traiter lesdits signaux électrophysiologiques ;
- un effecteur (5), configuré pour être actionné par un signal de commande généré par le processeur ;
- le processeur (3) étant configuré pour mettre en œuvre les étapes ii) à iv) du procédé objet de la revendication 6 ou de la revendication 7, à partir des signaux électrophysiologiques ($S_1...S_{I3}$) acquis par les capteurs.

## Patentansprüche

**1.** Verfahren zur Kalibrierung einer direkten neuronalen Schnittstelle (1), wobei die Schnittstelle geeignet ist, elektrophysiologische Signale ($S_1...S_{I3}$) zu erfassen und aus den erfassten elektrophysiologischen Signalen einen Eingangstensor $(\underline{\boldsymbol{X}})$ zu bilden, um mithilfe eines prädiktiven Modells einen Ausgangstensor $(\underline{\boldsymbol{Y}})$ zu erstellen, der dazu bestimmt ist, einen Effektor (5) zu steuern, wobei das prädiktive Modell durch eine Kalibrierung erhalten wird, wobei die Kalibrierung die folgenden Schritte umfasst:

a) Erfassung von elektrophysiologischen Kalibrierungssignalen, Eingangskalibrierungssignale genannt, um einen Tensor $(\underline{\boldsymbol{X}}^{(t)})$, Eingangskalibrierungstensor genannt, zu erhalten, wobei die elektrophysiologischen Kalibrierungssignale für eine neuronale Aktivität während der Kalibrierung repräsentativ sind;
b) Erfassung von Kalibrierungssignalen, Ausgangskalibrierungssignale genannt, um einen Tensor, Ausgangskalibrierungstensor **($\underline{\mathbf{Y}}^{(t)}$)** genannt, zu erhalten, wobei jedes Ausgangskalibrierungssignal für ein Signal zur Steuerung des Effektors (5) oder eines Zustands des Effektors (5) während der Kalibrierung repräsentativ ist;
c) Berechnung eines Kovarianztensors ($\underline{\underline{\mathbf{C}}}_{\mathbf{XX}}^{(t)}$), der eine Kovarianz des Eingangskalibrierungstensors ($\underline{\mathbf{X}}^{(t)}$) repräsentiert;
d) Berechnung eines Kreuzkovarianztensors ($\underline{\underline{\mathbf{C}}}_{\mathbf{XY}}^{(t)}$), der eine Kovarianz des Eingangskalibrierungstensors ($\underline{\mathbf{X}}^{(t)}$) und des Ausgangskalibrierungstensors ($\underline{\mathbf{Y}}^{(t)}$) repräsentiert;
e) Anwendung einer multivariaten Regression der partiellen kleinsten Quadrate, ausgehend von dem in Schritt c) erhaltenen Kovarianztensor und dem in Schritt d) erhaltenen Kreuzkovarianztensor, um ein prädiktives Modell $(\{\tilde{\underline{\mathbf{B}}}^{f}\}_{f=1}^{F\ t}, \{\underline{\mathbf{Y}}_{0}^{f}\}_{f=1}^{F\ t})$ sowie Kalibrierungsparameter $(\{\mathbf{w}_{1}^{f},\ldots\mathbf{w}_{k}^{f}\}_{f=1}^{F\ t})$ zu erhalten;

wobei die Schritte a) bis e) während einer aktuellen Kalibrierungsperiode ($t$) ausgeführt werden, wobei das Verfahren so beschaffen ist, dass nach einer ersten Kalibrierungsperiode ($t$ = 1)

- der Schritt c) eine Berücksichtigung des Kovarianztensors ($\underline{\underline{\mathbf{C}}}_{\mathbf{XX}}^{(t-1)}$) umfasst, der während einer früheren Kalibrierungsperiode ($t$ - 1) berechnet und mit einem Vergessensfaktor ($\lambda$) gewichtet wurde;
- der Schritt d) eine Berücksichtigung des Kreuzkovarianztensors ($\underline{\underline{\mathbf{C}}}_{\mathbf{XY}}^{(t-1)}$) umfasst, der während der früheren Kalibrierungsperiode ($t$ - 1) berechnet und mit dem Vergessensfaktor ($\lambda$) gewichtet wurde;
- der Schritt e) eine Berücksichtigung von Kalibrierungsparametern $(\{\mathbf{w}_{1}^{f},\ldots\mathbf{w}_{k}^{f}\}_{f=1}^{F\ t-1})$ umfasst, die aus der früheren Kalibrierungsperiode resultieren.

**2.** Verfahren nach Anspruch 1, wobei der Vergessensfaktor ($\lambda$) eine positive reelle Zahl ist, die streng zwischen 0 und 1 liegt.

**3.** Verfahren nach Anspruch 1 oder Anspruch 2, wobei in Schritt e) die Kalibrierungsparameter einen Satz von Projektionsvektoren $(\{\mathbf{w}_{1}^{f},\ldots\mathbf{w}_{k}^{f}\}_{f=1}^{F\ t})$ umfassen, derart, dass nach der ersten Kalibrierungsperiode Schritt e) eine Berücksichtigung eines Satzes von Projektionsvektoren $(\{\mathbf{w}_{1}^{f},\ldots\mathbf{w}_{k}^{f}\}_{f=1}^{F\ t-1})$ umfasst, die aus der früheren Kalibrierung resultieren.

**4.** Verfahren nach einem der vorhergehenden Ansprüche, wobei in Schritt e) die multivariate Regression der partiellen kleinsten Quadrate gemäß einer vordefinierten maximalen Anzahl (**F**) von Iterationen durchgeführt wird, wobei jeder Iteration ein Iterationsrang ($f$) entspricht, wobei jede Iteration ein prädiktives Modell ($\tilde{\underline{\mathbf{B}}}^{f}$) erzeugt, wobei das prädiktive Modell ermöglicht, einen Tensor ($\hat{\underline{\mathbf{Y}}}^{f}$), Ausgangstensor genannt, anhand eines Eingangstensors ($\underline{\mathbf{X}}$) zu schätzen,

- wobei der Eingangstensor ($\underline{\mathbf{X}}$) aus elektrophysiologischen Signalen gebildet wird, die zu unterschiedlichen Zeitpunkten erfasst wurden;
- wobei der Ausgangstensor ($\hat{\underline{\mathbf{Y}}}^{f}$) Ausgangsdaten umfasst, die dazu bestimmt sind, Steuersignale zum Steuern des Effektors (5) zu bilden.

**5.** Verfahren nach Anspruch 4, welches eine Bestimmung eines sogenannten optimalen Iterationsranges ($F^*$) umfasst, wobei die Bestimmung gemäß den folgenden Schritten durchgeführt wird:

- Berücksichtigung eines Eingangskalibrierungstensors ($\underline{\mathbf{X}}^{(t)}$) und eines Ausgangskalibrierungstensors ($\underline{\mathbf{Y}}^{(t)}$, die der aktuellen Kalibrierungsperiode ($t$) entsprechen;
- Berücksichtigung der prädiktiven Modelle $(\{\tilde{\underline{\mathbf{B}}}^{f}\}_{f=1}^{F\ t-1})$, die jeweils verschiedenen Iterationsrängen ($f$) der multivariaten Regression der partiellen kleinsten Quadrate, die während der früheren Kalibrierungsperiode ($t$ - 1 ) durchgeführt wurde, zugeordnet sind;
- Anwendung jedes prädiktiven Modells ($\tilde{\underline{\mathbf{B}}}^{f(t-1)}$) auf den Eingangskalibrierungstensor der aktuellen Kalibrierungsperiode ($\underline{\mathbf{X}}^{(t)}$), um eine Schätzung ($\hat{\underline{\mathbf{Y}}}^{f(t)}$) des Ausgangskalibrierungstensors der aktuellen Kalibrierungsperiode zu erhalten, wobei jede Schätzung dem Iterationsrang ($f$) zugeordnet ist, der dem prädiktiven Modell entspricht, anhand dessen die Schätzung vorgenommen wird;
- Vergleich, für jeden Iterationsrang ($f$), jeder Schätzung ($\underline{\hat{Y}}^{f(t)}$), die im vorhergehenden Schritt erhalten wurde, mit dem Ausgangskalibrierungstensor der aktuellen Kalibrierungsperiode ($\underline{Y}^{(t)}$);
- Bestimmung eines sogenannten optimalen Iterationsranges ($F^*$) in Abhängigkeit von jedem Vergleich.

**6.** Verfahren zur Steuerung eines Effektors durch eine direkte neuronale Schnittstelle, welches die folgenden Schritte umfasst:

i) Erfassung elektrophysiologischer Signale ($S_1..S_{l3}$), die in der Großhirnrinde eines Individuums erzeugt wurden;
ii) Verarbeitung der elektrophysiologischen Signale, um Eingangsdaten zu bilden, die gemäß einem Eingangstensor ($\underline{X}$) zusammengeführt werden;
iii) Anwendung eines prädiktiven Modells ($\tilde{\underline{B}}^f, \tilde{\underline{B}}^{f=F^*}$) auf den Eingangstensor **($\underline{X}$)**, um einen Ausgangstensor ($\underline{\hat{Y}}^f, \underline{\hat{Y}}^{f=F^*}$) zu schätzen,
iv) Bildung eines Signals zur Steuerung des Effektors (5) aus dem Ausgangstensor;

wobei das Verfahren **dadurch gekennzeichnet ist, dass** in Schritt iii) das prädiktive Modell durch eine Kalibrierung bestimmt wird, die gemäß einem Verfahren zur Kalibrierung nach einem der Ansprüche 1 bis 5 durchgeführt wird.

**7.** Verfahren zur Steuerung nach Anspruch 6, wobei in Schritt iii) das prädiktive Modell ($\tilde{\underline{B}}^{f=F^*}$) angewendet wird, das dem optimalen Iterationsrang ($F^*$) entspricht, der gemäß dem Verfahren zur Kalibrierung nach Anspruch 5 bestimmt wurde.

**8.** Direkte neuronale Schnittstelle (1), welche aufweist:

- Sensoren (2), die geeignet sind, elektrophysiologische Signale ($S_1...S_{l3}$) zu erfassen, die für eine kortikale Aktivität repräsentativ sind;
- einen Prozessor (3), um die elektrophysiologischen Signale zu verarbeiten;
- einen Effektor (5), der dafür ausgelegt ist, durch ein von dem Prozessor erzeugtes Steuersignal betätigt zu werden;
- wobei der Prozessor (3) dafür ausgelegt ist, die Schritte iii) bis iv) des Verfahrens nach Anspruch 6 oder nach Anspruch 7 ausgehend von den elektrophysiologischen Signalen ($S_1...S_{l3}$) auszuführen, die von den Sensoren erfasst wurden.

## Claims

**1.** A method for calibrating a direct neural interface (1), the interface being able to acquire electrophysiological signals ($S_1...S_{l3}$) and to form an input tensor **($\underline{X}$)**, on the basis of the electrophysiological signals acquired, so as to establish, with the aid of a predictive model, an output tensor ($\underline{Y}$), intended to control an effector (5), the predictive model being obtained by calibration, the calibration comprising the following steps:

a) acquisition of so-called input electrophysiological calibration signals to obtain a so-called input calibration tensor **($\underline{X}^{(t)}$)**, the electrophysiological calibration signals being representative of neural activity during calibration;
b) acquisition of so-called output calibration signals to obtain an output calibration tensor, so-called ($\underline{Y}^{(t)}$), each output calibration signal being representative of a control signal for the effector (5) or of a state of the effector (5) during calibration;
c) calculation of a covariance tensor ($\underline{C_{XX}}^{(t)}$), representing a covariance of the input calibration tensor **($\underline{X}^{(t)}$);**
d) calculation of a cross-covariance tensor ($\underline{C_{XY}}^{(t)}$) representing a covariance of the input calibration tensor **($\underline{X}^{(t)}$)** and of the output calibration tensor ($\underline{Y}^{(t)}$);
e) application of a partial least squares multivariate regression on the basis of the covariance tensor obtained in step c) and of the cross-covariance tensor obtained during step d) so as to obtain a predictive model $(\{\tilde{\underline{B}}^f\}_{f=1}^{F}{}^{t}, \{\underline{Y}_0^f\}_{f=1}^{F}{}^{t})$, as well as calibration parameters $(\{\boldsymbol{w}_1^f, \ldots \boldsymbol{w}_k^f\}_{f=1}^{F}{}^{t})$;

steps a) to e) being implemented during a current calibration period (t), the method being such that after a first calibration period ($t$ = 1),

- step c) comprises taking into account of the covariance tensor ($\underline{C_{XX}}^{(t-1)}$) calculated in the course of an earlier calibration period ($t$ - 1), and weighted by a forget factor ($\lambda$);
- step d) comprises taking into account of the cross-covariance tensor ($\underline{C_{XY}}^{(t-1)}$) calculated in the course of the earlier calibration period ($t$ - 1), and weighted by the forget factor ($\lambda$);
- step e) comprises taking into account of calibration parameters $\left(\left\{w_1^f, \ldots w_k^f\right\}_{f=1}^{F}{}^{t-1}\right)$ resulting from the earlier calibration period.

**2.** The method as claimed in claim 1, in which the forget factor ($\lambda$) is a positive real lying strictly between 0 and 1.

**3.** The method as claimed in claim 1 or claim 2, in which during step e), the calibration parameters comprise a collection of projection vectors $\left(\left\{w_1^f, \ldots w_k^f\right\}_{f=1}^{F}{}^{t}\right)$, so that after the first calibration period, step e) comprises a taking into account of a collection of projection vectors $\left(\left\{w_1^f, \ldots w_k^f\right\}_{f=1}^{F}{}^{t-1}\right)$ resulting from the earlier calibration.

**4.** The method as claimed in any one of the preceding claims, in which during step e) the partial least squares multivariate regression is performed according to a predetermined maximal number of iterations, to each iteration there corresponding an iteration rank ($f$), each iteration generating a predictive model **($\tilde{\boldsymbol{B}}^f$),** the predictive model making it possible to estimate a tensor **($\hat{\boldsymbol{Y}}^f$),** so-called output tensor, on the basis of an input tensor ($\underline{\boldsymbol{X}}$),

- the input tensor ($\underline{\boldsymbol{X}}$) being formed on the basis of electrophysiological signals acquired at various instants;
- the output tensor ($\underline{\hat{\boldsymbol{Y}}^f}$) comprising output data intended to form control signals to control the effector (5).

**5.** The method as claimed in claim 4, comprising a determination of a so-called optimal iteration rank ($F^*$), the determination being carried out according to the following steps:

- taking into account of an input calibration tensor ($\underline{\boldsymbol{X}}^{(t)}$) and of an output calibration tensor ($\underline{\boldsymbol{Y}}^{(t)}$) corresponding to the current calibration period ($t$);
- taking into account of the predictive models $\left(\left\{\tilde{\boldsymbol{B}}^f\right\}_{f=1}^{F}{}^{t-1}\right)$ respectively associated with various iteration ranks ($f$) of the partial least squares multivariate regression performed during the earlier calibration period ($t$ - 1);
- application of each predictive model ($\tilde{\boldsymbol{B}}^{f(t-1)}$) to the input calibration tensor of the current calibration period ($\underline{\boldsymbol{X}}^{(t)}$), so as to obtain an estimation ($\underline{\hat{\boldsymbol{Y}}}^{f(t)}$) of the output calibration tensor of the current calibration period, each estimation being associated with the rank of the iteration ($f$) corresponding to the predictive model on the basis of which the estimation is performed;
- comparison, for each iteration rank ($f$), of each estimation ($\underline{\hat{\boldsymbol{Y}}}^{f(t)}$) obtained during the previous step, with the output calibration tensor of the current calibration period ($\underline{\boldsymbol{Y}}^{(t)}$);
- determination of a, so-called optimal, iteration rank ($F^*$) as a function of each comparison.

**6.** A method of controlling an effector through a direct neural interface, comprising the following steps:

i) acquisition of electrophysiological signals ($S_1 \ldots S_{I_3}$) produced in the cortex of an individual;
ii) processing of the electrophysiological signals, to form input data, assembled according to an input tensor ($\underline{\boldsymbol{X}}$);
iii) application of a predictive model **($\tilde{\boldsymbol{B}}^f, \tilde{\boldsymbol{B}}^{f=F^*}$)** to the input tensor ($\underline{\boldsymbol{X}}$) so as to estimate an output tensor ($\underline{\hat{\boldsymbol{Y}}}^f \underline{\hat{\boldsymbol{Y}}}^{f=F^*}$);
iv) formation of a control signal for the effector (5) on the basis of the output tensor;

the method being **characterized in that** during step iii) the predictive model is determined by a calibration carried out according to a calibration method which is the subject of any one of claims 1 to 5.

**7.** The control method as claimed in claim 6, in which during step iii), the predictive model ($\hat{\boldsymbol{B}}^{f=F^*}$) corresponding to the optimal iteration rank ($F^*$) determined according to the calibration method according to claim 5 is applied.

**8.** A direct neural interface (1), comprising:

- sensors (2), able to acquire electrophysiological signals ($S_1 ... S_{I_3}$) representative of a cortical activity;
- a processor (3), for processing said electrophysiological signals;
- an effector (5), configured to be actuated by a control signal generated by the processor;
- the processor (3) being configured to implement steps ii) to iv) of the method which is the subject of claim 6 or of claim 7, on the basis of the electrophysiological signals ($S_1 ... S_{I_3}$) acquired by the sensors.

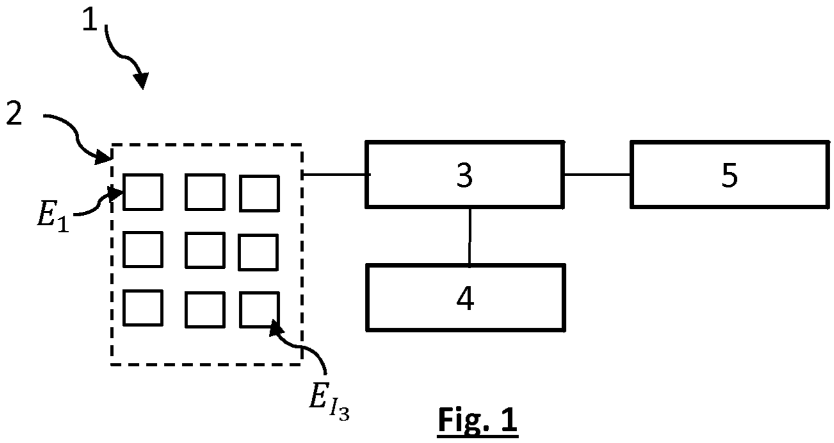
1
2
$E_1$
$E_{I_3}$
3
4
5

**Fig. 1**

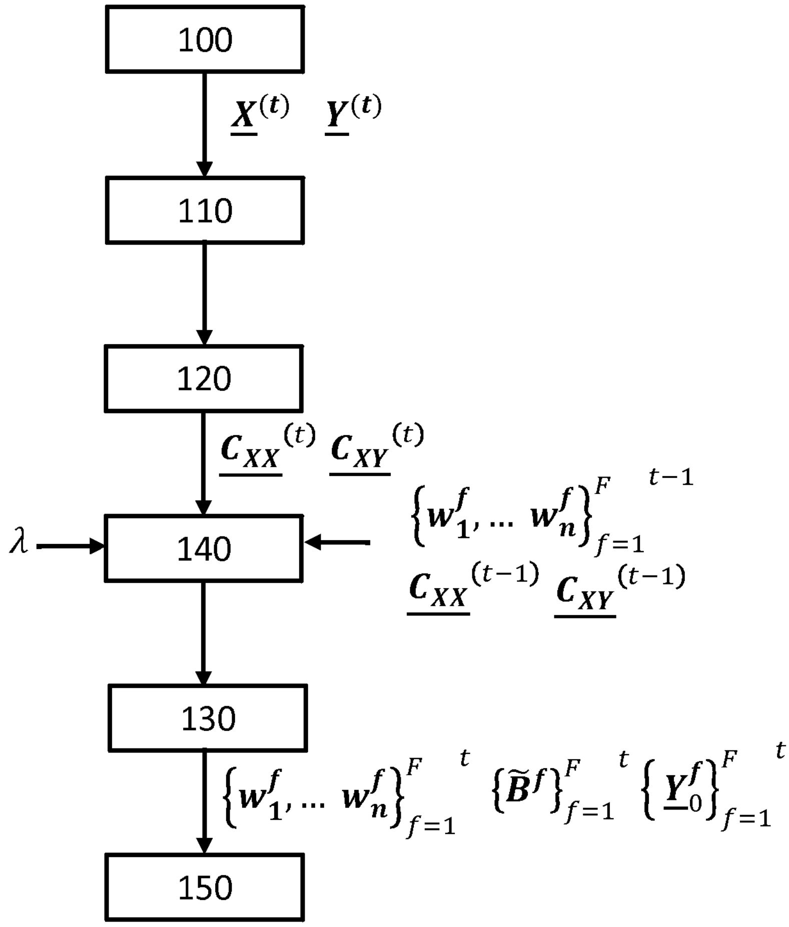
100
$\underline{X}^{(t)}$ $\underline{Y}^{(t)}$
110
120
$\underline{C_{XX}}^{(t)}$ $\underline{C_{XY}}^{(t)}$
$\lambda$
140
$\left\{w_1^f, \dots w_n^f\right\}_{f=1}^{F\ \ t-1}$
$\underline{C_{XX}}^{(t-1)}$ $\underline{C_{XY}}^{(t-1)}$
130
$\left\{w_1^f, \dots w_n^f\right\}_{f=1}^{F\ \ t}$ $\left\{\widetilde{B}^f\right\}_{f=1}^{F\ \ t}$ $\left\{\underline{Y}_0^f\right\}_{f=1}^{F\ \ t}$
150

**Fig. 2A**

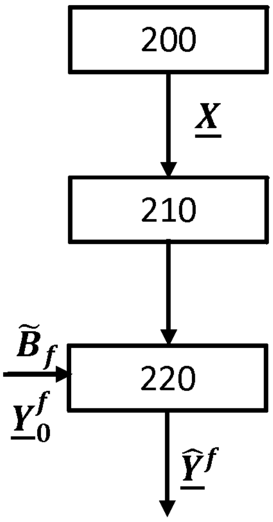


**Fig. 2B**

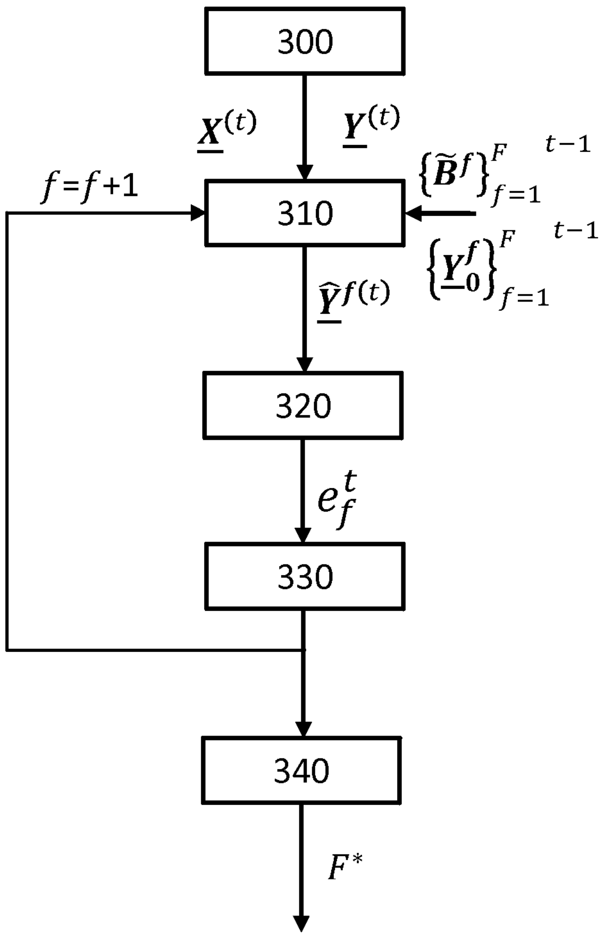


**Fig. 2C**

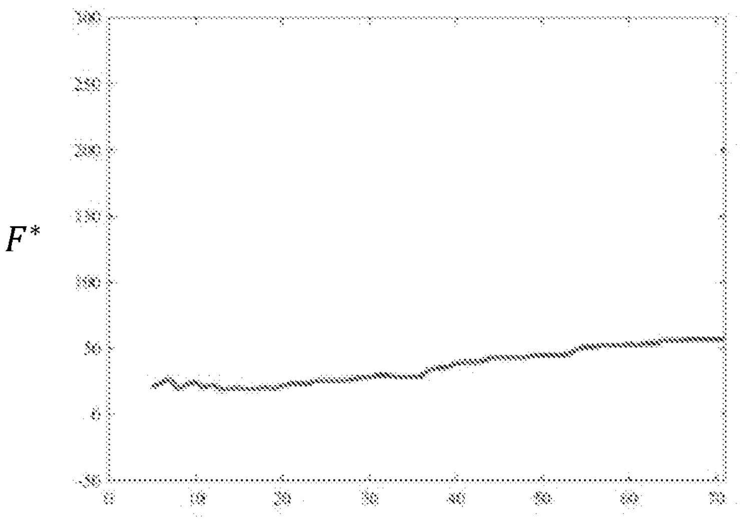


**Fig. 3**

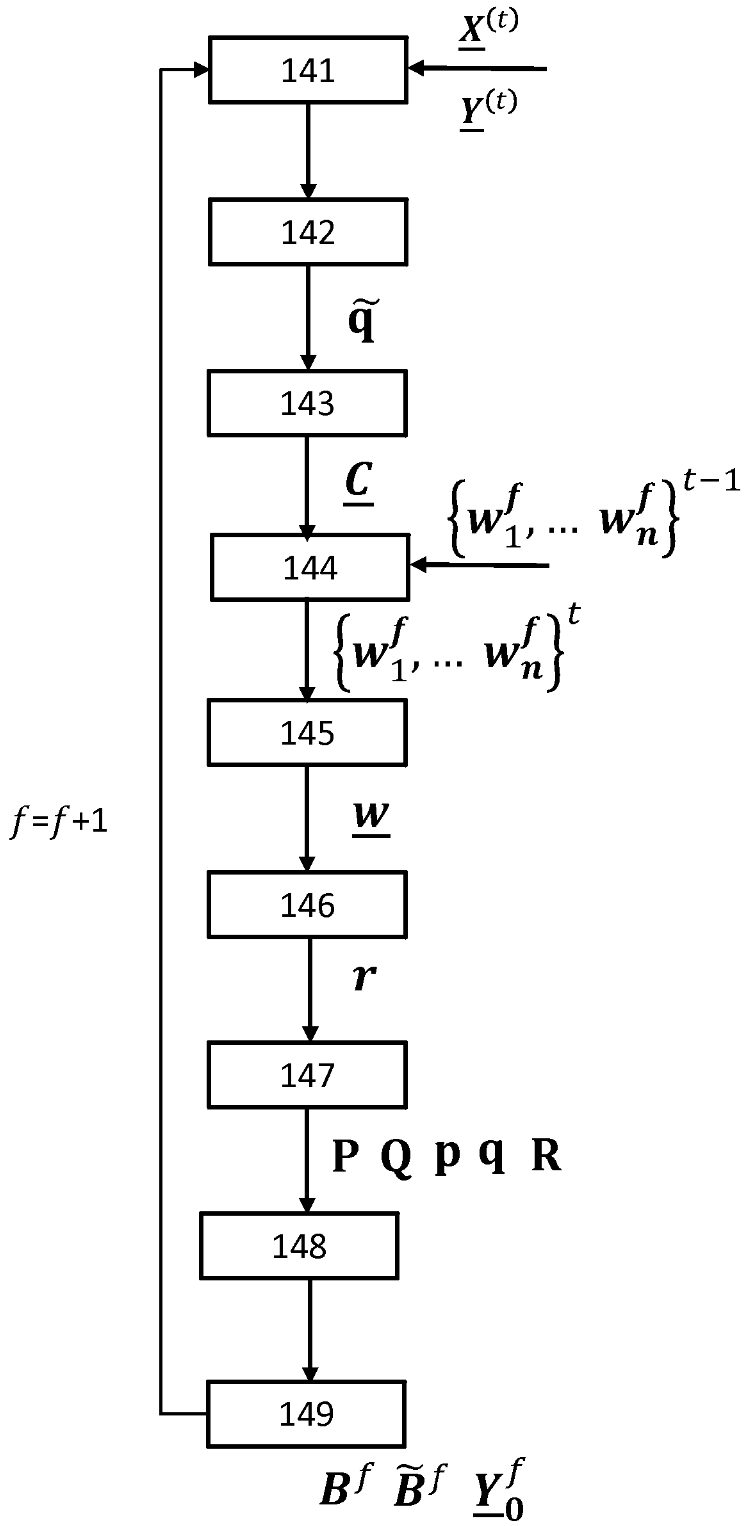
141
$\underline{X}^{(t)}$
$\underline{Y}^{(t)}$
142
$\tilde{q}$
143
$\underline{C}$
144
$\{w_1^f, \dots w_n^f\}^{t-1}$
$\{w_1^f, \dots w_n^f\}^{t}$
145
$\underline{w}$
146
$r$
147
P Q p q R
148
149
$B^f$ $\tilde{B}^f$ $\underline{Y}_0^f$
$f=f+1$

Fig. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 9480583 B


### Littérature non-brevet citée dans la description


- **ELISEYEV A ; AKSENOVA T.** Recursive N-way Partial Least Squares for Brain Computer Interface. *PlOS ONE,* Juillet 2013, vol. 8 (7), e69962
- **YELISYEYEV A.** Brain-Computer Interface with cortical electrical activity recording. *Human health and pathology*
- **DAYAL B.** Improved PLS Algorithms. *Journal of Chemometrics,* 1997, vol. 11, 73-85